# EUROPÄISCHE PATENTSCHRIFT

(11) **EP 0 844 994 B1**
(45) Veröffentlichungstag und Bekanntmachung des Hinweises auf die Patenterteilung: **12.05.1999**
(21) Anmeldenummer: 96927693.0
(22) Anmeldetag: 06.08.1996
(51) Int. Cl.: C07D 307/32

(54) **VERFAHREN ZUR HERSTELLUNG VON BUTYROLACTONEN**
BUTYROLACTONE-PREPARATION PROCESS
PROCEDE DE PREPARATION DE BUTYROLACTONES

(30) Priorität: 19.08.1995 DE 19530549; 29.11.1995 DE 19544408
(43) Veröffentlichungstag der Anmeldung: 03.06.1998
(73) Patentinhaber: BASF AKTIENGESELLSCHAFT, 67056 Ludwigshafen (DE)
(72) Erfinder: HEIDER, Marc, D-67433 Neustadt (DE); RÜHL, Thomas, D-67227 Frankenthal (DE); HENKELMANN, Jochem, D-68165 Mannheim (DE); STUTZ, Susanne, D-69469 Weinheim (DE); PREISS, Thomas, D-67065 Ludwigshafen (DE); RÜTTER, Heinz, D-67126 Hochdorf-Assenheim (DE); SCHÄFER, Martin, D-67063 Ludwigshafen (DE); HÖHN, Arthur, D-67281 Kirchheim (DE)
(74) Vertreter: Isenbruck, Günter, Dr.
(86) Internationale Anmeldenummer: EP9603472
(87) Internationale Veröffentlichungsnummer: WO9707111

(56) Entgegenhaltungen:
- CHEMICAL ABSTRACTS, vol. 115, no. 5, 5.August 1991 Columbus, Ohio, US; abstract no. 49276b, T. JOH ET AL.: "Rhodium-catalyzed carbonylation of acetylenes under water-gas shift reaction conditions. Selective synthesis of furan-2(5H)-ones." Seite 804; Spalte 2; XP002019901 in der Anmeldung erwähnt & ORGANOMETALLICS, Bd. 10, Nr. 7, 1991, Seiten 2493-2498,
- CHEMICAL ABSTRACTS, vol. 121, no. 9, 29.August 1994 Columbus, Ohio, US; abstract no. 108410q, T. JOH ET AL.: "Rhodium-catalyzed synthesis of 2(5H)-furanones from terminal alkynes and non-substituted alkynes under water-gas shift reaction conditions" Seite 1034; Spalte 2; XP002019902 in der Anmeldung erwähnt & INORG. CHIM. ACTA, Bd. 220, Nr. 1-2, 1994, Seiten 45-53,
- DATABASE WPI Week 8818 Derwent Publications Ltd., London, GB; AN 88-123681 XP002019903 & JP 63 068 580 A (SEKISUI CHEM IND KK) , 11.September 1986 in der Anmeldung erwähnt & JP 63 068 580 A (...) 11.September 1986

## Beschreibung

Die Erfindung betrifft ein Verfahren zur Herstellung von Butyrolactonen durch Umsetzung von Acetylenen mit Kohlenmonoxid und Wasserstoffgas oder in situ gebildetem Wasserstoff in Gegenwart von Übergangsmetallkatalysatoren über die Stufe der entsprechenden 2-(5H)-Furanone, sowie ein verbessertes Verfahren zur Herstellung dieser Furanone, die auch in einer getrennten Hydrierstufe in die Butyrolactone umgewandelt werden können.

γ-Butyrolacton ist ein bedeutendes Produkt der chemischen Industrie. Es dient als Zwischenprodukt für die Herstellung von Pyrrolidon und dessen Derivaten sowie als Lösungsmittel.

Butyrolacton wird industriell durch verschiedene mehrstufige Verfahren hergestellt. So führt die Dehydrocyclisierung von 1,4-Butandiol in hohen Ausbeuten zu Butyrolacton. Alternativ kann Maleinsäureanhydrid partiell zu Butyrolacton hydriert werden. Beide Ausgangsverbindungen für die Herstellung von γ-Butyrolacton sind nur in mehrstufigen Prozessen aus Basischemikalien zugänglich (Weissermel, Arpe, Industrielle Organische Chemie, 2. Auflage 1978, Verlag Chemie, S. 97).

Die Umsetzung von Alkinen und CO in Gegenwart von Rhodiumkatalysatoren ist für die Herstellung von ungesättigten 2(5H)-Furanonen, die als Zwischenprodukte für die Herstellung von Arzneimitteln dienen, beschrieben worden (Joh et al., Inorg. Chim. Acta, 220 (1994) 45; Organometallics 10 (1991) 2493; JP-A 88/68580).

Es bestand nun die Aufgabe, ein Verfahren bereitzustellen, das ausgehend von einfachen Grundchemikalien wie Alkinen und CO, einstufig oder höchstens 2-stufig zu Butyrolactonen führt. Weiterhin bestand die Aufgabe, in 3- bzw. 4-Stellung substituierte Butyrolactone bereitzustellen, die als Zwischenprodukte in der Feinchemie von Interesse sind.

Schließlich bestand die Aufgabe, das Verfahren zur Herstellung von 2(5H)-Furanonen, aus dem in einer zweiten Stufe durch Hydrierung Butyrolactone hergestellt werden können, so zu verbessern, daß eine technische Herstellung möglich ist.

Die Aufgabe wurde erfindungsgemäß gelöst mit einem Verfahren zur Herstellung von Butyrolactonen der allgemeinen Formel I 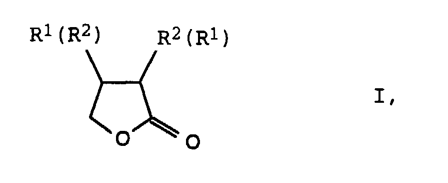 in der die Substituenten R¹ und R² für Wasserstoff, Alkyl-, Hydroxyalkylgruppen mit vorzugsweise 1 bis 8 C-Atomen oder für gegebenenfalls inerte Substituenten tragende Aryl- und Trialkylsilylgruppen stehen, indem man ein Alkin der allgemeinen Formel II

R¹―C≡C―R² II

in der die Substituenten die oben angegebene Bedeutung haben, mit Kohlenmonoxid und Wasserstoffgas oder mit in situ gebildetem Wasserstoff in Gegenwart eines Übergangsmetallkatalysators unter erhöhtem Druck und erhöhter Temperatur umsetzt. In situ gebildeter Wasserstoff wird dabei vorteilhaft mit CO und Wasser über das Wassergasgleichgewicht CO + H₂O 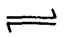 H₂+CO₂ erzeugt.

Es war dabei überraschend, daß es gelingt, in einer Stufe Butyrolactone herzustellen, d.h. daß die als Zwischenprodukte entstehen 2(5H)-Furanone unter den Reaktionsbedingungen zu den Butyrolactonen weiterreagieren.

Alternativ kann auch die an sich bekannte Herstellung von 2-(5H)-Furanonen der allgemeinen Formel III 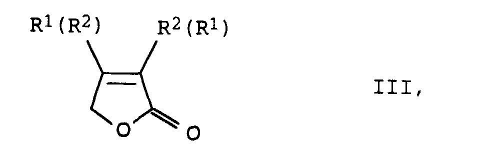 in der die Substituenten R¹ und R² für Wasserstoff, Alkyl, Hydroxylalkyl-, gegebenenfalls inerte Substituenten tragende Aryl- oder Trialkylsilylgruppen stehen, dadurch verbessert werden, daß man Alkine der allgemeinen Formel II

R¹―C≡C―R² II,

in denen R¹ und R² die oben genannte Bedeutung haben, mit Kohlenmonoxid und in situ gebildetem Wasserstoff aus Kohlenmonoxid und Wasser oder mit Wasserstoffgas in Gegenwart von Übergangsmetall-Katalysatoren, Aminbasen und Halogeniden bei Temperaturen von 60 - 140°C und Gesamtdrücken von 20 - 350 bar umsetzt.

Obgleich Takashi Joh et al. in Organometallics 1991, 10, S. 2494 linke Spalte, erster Absatz beschreiben: "The use of hydrogen gas as a hydrogen source in place of water gave hydroxymethylated product 4 and stilben 5, and no, furanone was obtained", wurde überraschenderweise ferner gefunden, daß man Furanone der oben genannten Formel I sogar ohne die Notwendigkeit von einem Halogenidzusatz erhält, wenn man die Umsetzung bei einem Wasserstoffpartialdruck von mehr als 50 bar durchführt.

Man kann dann die so in deutlich höheren Raum-Zeit-Ausbeuten erhalten 2(5H)-Furanone auch in einem zweiten Schritt getrennt zu den entsprechenden Butyrolactonen hydrieren. Die Hydrierung kann dabei in an sich bekannter Weise kontinuierlich oder in Batch-Fahrweise mit an sich bekannten Hydrierkatalysatoren, z.B. den in Houben-Weyl, Band 4/2, für die Hydrierung von Kohlenstoff-Doppelbindungen genannten Katalysatoren durchgeführt werden. Bevorzugt sind solche Katalysatoren, die Metalle der VIII-Nebengruppe, insbesondere Rh, Ni oder Pd oder Gemische dieser Metalle als Aktivkomponenten enthalten.

Die Alkine der Formel II können gleiche oder verschiedene Substituenten tragen. Sind die Substituenten R¹ und R² voneinander verschieden, können sie im Reaktionsprodukt jeweils in 3- bzw. 4-Stellung eingebaut werden. In diesen Fällen sind also Isomerengemische zu erwarten. Aus diesem Grund sind in Formel I die Substituenten alternativ in 3- bzw. 4- Stellung gezeichnet. Die Formel steht somit sowohl für Verbindungen, die in 3-Position wie auch für Verbindungen, die in 4-Position den Substituenten R¹ tragen.

Die Alkine II können Alkylgruppen tragen, bevorzugt C₁-C₈-Alkylgruppen wie in Propin, 1-Butin, 2-Butin, 1-Hexin und 1-Octin. Weiterhin können sie Hydroxyalkylgruppen tragen, bevorzugt Hydroxy-C₁-C₄-alkylgruppen wie in 1-Butin-3-ol, 1,4-Butindiol und Propargylalkohol. Von den Arylgruppen-tragenden Alkinen sind Phenylalkine bevorzugt, z.B. Phenylacetylen und Diphenylacetylen. Die Arylgruppen können unter den Reaktionsbedingungen stabile Substituenten tragen wie Halogen, insbesondere Chlor, Alkoxy, insbesondere Methoxy, und Alkyl, insbesondere C₁-C₄-Alkyl. Weiterhin kommen Alkine in Betracht, die Trialkylsilylgruppen tragen, z.B. Trimethylsilylacetylen.

Bevorzugt sind solche Alkine, in denen mindestens einer der Substituenten R¹ und R² für Wasserstoff steht. Acetylen ist besonders bevorzugt.

Die Umsetzung des Alkins zum Butyrolacton kann in zwei technischen Varianten ausgeführt werden. Die eine Ausführungsform erfordert die Gegenwart von Wasserstoffgas und ist bevorzugt (Verfahren A):

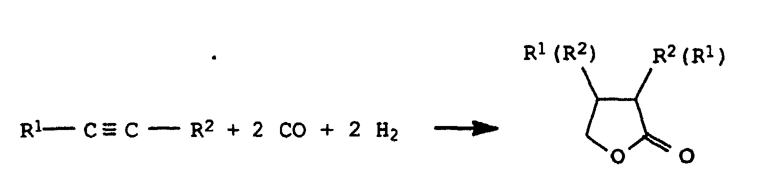

Die andere Variante erfordert die Gegenwart von Wasser (Verfahren

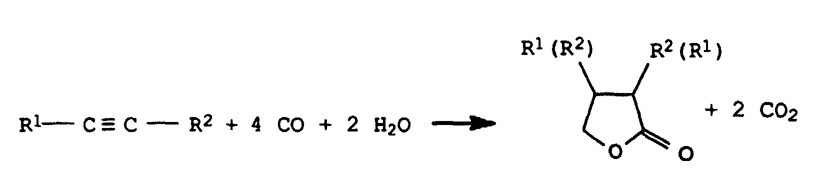

Aufgrund der verschiedenen Bruttogleichung ist es empfehlenswert, in Verfahren A mindestens 2 Äquivalente, in Verfahren B mindestens 4 Äquivalente CO je Äquivalent Alkin zu verwenden. CO kann auch im Überschuß eingesetzt werden, wobei jedoch in der Regel nicht mehr als ein 50facher Überschuß, bezogen auf das Alkin, an CO verwendet werden sollte, da noch größere Überschüsse keine erkennbaren technischen Vorteile bringen.

Es ist auch möglich die Varianten A und B zu kombinieren.

In Verfahren A werden bevorzugt 2 bis 50 Äquivalente Wasserstoff H₂ je Äquivalent Alkin eingesetzt. In einer besonders bevorzugten Ausführungsform wird der Wasserstoff zusammen mit CO in Form von Synthesegas verwendet.

In Verfahren B wird das Alkin der Formel II mit CO und Wasser umgesetzt. Bevorzugt werden 2 bis 50 Äquivalente Wasser je Äquivalent Alkin eingesetzt.

Die erfindungsgemäße Herstellung von Butyrolactonen der Formel I erfolgt in Gegenwart von Übergangsmetallkatalysatoren. Prinzipiell können alle Katalysatoren eingesetzt werden, die in der Lage sind, das Wassergasgleichgewicht gemäß der folgenden Gleichung einzustellen (water-gas shift catalysts; s. Parshall et al., Homogeneous Catalysis, Wiley, 2. Auflage 1992, Kapitel 5.7):

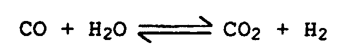

Demgemäß bedeutet der Begriff "in situ gebildeter Wasserstoff", daß sich das Wassergasgleichgewicht einstellt und daraus Wasserstoff zur Verfügung steht.

Sowohl Homogen- wie auch Heterogenkatalysatoren können verwendet werden. Als aktive Metallverbindungen kommen Verbindungen des Rhodiums, Iridiums, Rutheniums, Osmiums, Palladiums, Platins, Eisens, Nickels, Kupfers und Cobalts sowie die Metalle selbst in Betracht.

Als Heterogenkatalysatoren können die genannten Metalle bzw. ihre Verbindungen auf inerten Trägern wie Kohle, Aluminiumoxid, Siliciumdioxid und Zirkoniumdioxid verwendet werden. Solche Katalysatoren sind im Handel erhältlich oder nach bekannten Methoden herstellbar, beispielsweise durch Tränkung inerter Träger mit Lösungen der Metallverbindungen und Calcinierung.

Beispielhaft seien Pd/Kohle und Pd/Aluminiumoxid genannt.

Die Reaktion kann in der Gasphase durchgeführt werden, bevorzugt wird jedoch eine Flüssigphasenumsetzung.

Bevorzugt sind Homogenkatalysatoren. Die aktiven Metalle können in Form von Halogeniden, Acetaten, Nitraten, Oxiden, Acetylacetonaten und bevorzugt Carbonylen verschiedener Wertigkeitsstufen eingesetzt werden, wobei sich die aktive Verbindung im Reaktionsgemisch unter den Reaktionsbedingungen bildet.

Ruthenium- und Nickelverbindungen sind im erfindungsgemäßen Verfahren bevorzugt, besonders bevorzugt sind aber Rhodiumverbindungen. Letzteres ist überraschend, da gemäß JA N. 3-94238 (Application Number) für die Herstellung der als Zwischenprodukte zu postulierenden Furanone Rutheniumkatalysatoren bessere Ergebnisse als Rhodiumkatalysatoren liefern sollen.

Im folgenden werden beispielhaft einige Übergangsmetallverbindungen genannt, die als erfindungsgemäße Katalysatoren oder Vorstufen von Katalysatoren, aus denen sich die katalytisch aktive Verbindung unter Reaktionsbedingungen bildet, in Betracht kommen:
Rh₆(CO)₁₆, Rh₄(CO)₁₂, Rh₂O₃, RhCl₃·3H₂O, RhCl(PPh₃)₃ (Ph steht für Phenyl), (codRhCl)₂ (cod steht für 1,5-Cyclooctadien), HRh(CO)(PPh₃)₃;
RuCl₃, Ru(acac)₃ (acac steht für Acetylacetonat), Ru₃(CO)₁₂, [CpRu(CO)₂]₂ (Cp steht für Cyclopentadienyl);
NiCl₂, NiBr₂, Ni(CO)₄, Ni(cod)₂, [Ni(NH₃)₆]Cl₂;
PtCl₂, PtBr₂, PtCl₄, PtO₂, [Pt(NH₃)₄]Cl₂·H₂O;
Pd(ac)₂ (ac steht für Acetyl), PdCl₂, PdBr₂, K₂[PdCl₄], K₂[PdCl₆], Pd(PPh₃)₄;
CuCl₂, CuBr₂, Cu(acac)₂, Cu(ac)₂, CuO, Cu₂O, CuI.

Die Menge an eingesetztem Katalysator liegt in der Regel bei 0,01 bis 10 mmol pro Mol Alkin.

Durch Zusatzstoffe läßt sich die Aktivität der Übergangsmetallkatalysatoren deutlich steigern. Zu diesen Verbindungen gehören Amine. Hierzu kommen primäre, sekundäre und tertiäre Alkylamine und Cycloalkylamine ebenso in Betracht wie stickstoffhaltige Heterocyclen, z.B. Methylamin, Ethylamin, Anilin, Diethylamin, Triethylamin, Tributylamin, Trioctylamin, Pyridin, Chinolin, Isochinolin und Dimethylaminopyridin. Weiterhin zeigen Ammoniumsalze wie Triethylammoniumhydrochlorid, Tetraethylammoniumchlorid, Tetrabutylammoniumacetat, Tetrabutylanmoniumnitrat und Tetrabutylammoniumhydroxid positive Effekte.

Die Aktivität der Katalysatoren und damit die Raum-Zeit-Ausbeute läßt sich durch Zusatz von Halogeniden erheblich steigern. Im einzelnen können Alkalimetall- und Erdalkalimetallhalogenide wie NaCl, NaBr, NaJ, KCl, KBr, KJ, CaCl₂, CaBr₂ und CaJ₂ sowie Halogenide mit organischen Kationen wie Tetramethylammoniumchlorid, Tetramethylammoniumbromid, Tetramethylamnoniumjodid, Tetrabutylammoniumchlorid, Tetrabutylammoniumbromid und Tetrabutylammoniumjodid eingesetzt werden. Bevorzugte Halogenide sind die Jodide.

Auch Polymerisationsinhibitoren für olefinisch ungesättigte organische Verbindungen wie Hydrochinonmonomethylether, 2,6-Di-tert.-butyl-4-methylphenol und Phenothiazin kommen als Zusatzstoffe in Betracht.

Die Menge an eingesetzten Zusatzstoffen ist in weiten Grenzen variabel und kann zwischen 0,1 und 10000 mol Zusatzstoff je Mol Katalysator liegen. Bevorzugt werden 0,5 bis 5 mol je Mol Katalysator eingesetzt. Es können ein oder mehrere Zusatzstoffe in einer Reaktion eingesetzt werden.

Die Reaktionstemperatur beträgt im allgemeinen 0 bis 300°C, bevorzugt 20 bis 200°C und besonders bevorzugt 50 bis 150°C. Der Druck beträgt in der Regel 20 bis 300 bar. Für Verfahren A ist ein Druck von 170 bis 280, für Verfahren B von 70 bis 280 bar bevorzugt. Die Reaktionszeit beträgt im allgemeinen 0,1 bis 24h, bevorzugt 0,5 bis 5h.

Die optimalen Reaktionsbedingungen können je nach gewähltem Katalysator und nach der eingesetzten Katalysatormenge variieren. Es hat sich als nützlich erwiesen, in den Fällen, in denen größere Mengen des entsprechenden 2(5H)-Furanons anstelle des gewünschten Butyrolactons der Formel I anfallen, die Reaktionsbedingungen zu verschärfen, was insbesondere durch eine Temperaturerhöhung erreicht werden kann. Der Fachmann kann die geeigneten Bedingungen nach wenigen orientierenden Vorversuchen leicht ermitteln.

Allgemein gilt, daß in der Regel Gemische aus Furanonen und Butyrolactonen entstehen, wobei bei milden Reaktionsbedingungen überwiegend Furanone und bei verschärften Bedingungen mit stark hydrierend wirkenden Katalysatoren überwiegend Butyrolactone gebildet werden. Demgemäß gibt es keine scharfe Grenze der Furanon- und Butyrolactonbildung. Wird z.B. Halogenid mitverwendet, bilden sich gemäß Verfahren A bereits bei milden Temperaturen von ca. 80 - 100°C überwiegend Butyrolactone mit hohen Raum-Zeit-Ausbeuten.

Es kann demgemäß sinnvoll sein, nur Gemische mit überwiegend Butyrolacton herzustellen. Die Gemische können dann entweder direkt der Hydrierung zugeführt werden oder in ihre Einzelkomponenten, Butyrolacton und Furanon, aufgetrennt werden. Das Furanon kann in die Umsetzung zurückgeführt oder getrennt hydriert werden.

Die Reaktion kann in der Gasphase und bevorzugt in der Flüssigphase durchgeführt werden. In Flüssigphase können unter den Reaktionsbedingungen inerte organische Lösungsmittel zugesetzt werden, beispielsweise Alkanole wie Methanol, Ethanol und Isopropanol, Ether wie Diethylether, Methyl-tert.-butylether, Tetrahydrofuran und 1,4-Dioxan, Ketone wie Aceton, Ester wie Essigsäuremethylester und Essigsäureethylester, weiterhin aromatische Kohlenwasserstoffe wie Benzol und Toluol, aliphatische Kohlenwasserstoffe wie Pentan und Hexan sowie polare, aprotische Lösungsmittel wie N-Methylpyrrolidon. Die Mengen können 5 bis 95 Gew.-% Lösungsmittel, bezogen auf den Reaktionsansatz, betragen.

Das erfindungsgemäße Verfahren kann in druckfesten Reaktoren wie Rühr-, Rohr- oder Schlaufenreaktoren kontinuierlich oder diskontinuierlich ausgeführt werden.

Bei diskontinuierlicher Fahrweise können das Alkin der Formel II, der Katalysator sowie gegebenenfalls ein Lösungsmittel und Zusatzstoffe im Reaktor vorgelegt werden. Bei Reaktionen mit Acetylen wird der entsprechende Acetylendruck eingestellt. In Verfahren B kann weiterhin die benötigte Menge Wasser in den Reaktionsansatz gegeben werden. Dann kann mit CO der Reaktionsdruck eingestellt werden. Anschließend wird in Verfahren A Wasserstoff bis zum gewünschten Druck zugefügt, sofern nicht von vornherein Synthesegas eingesetzt wird. Nach Beendigung der Umsetzung wird entspannt, wobei freigesetzte Gase für Folgereaktionen verwendet werden können. Die Aufarbeitung der Reaktionsausträge erfolgt nach bekannten Methoden, bevorzugt destillativ. Lösungsmittel, Katalysator und Zusatzstoffe können nach ihrer Aufarbeitung ebenfalls in weiteren Reaktionen wiederverwendet werden.

In einer bevorzugten kontinuierlichen Ausführungsform kann bei der Umsetzung Acetylen in einem Sättiger in einem Lösungsmittel gelöst werden. Der Katalysator, gegebenenfalls Wasser sowie Zusatzstoffe werden ebenfalls gelöst. Diese Lösung wird auf den Reaktionsdruck verdichtet und in den Reaktor gepumpt. CO, weiteres Acetylen und gegebenenfalls Wasserstoff werden über eine Gasdüse in den Reaktor eingebracht. Der Reaktionsaustrag wird entspannt, die flüssige Phase wird destillativ aufgearbeitet und der Katalysator wird in den Reaktionskreislauf zurückgeführt. Auch die Gasphase kann - gegebenenfalls nach einem Reinigungsschritt - zurück in den Reaktor geführt werden.

### Beispiele

### Beispiel 1 (Verfahren A)

In 135 ml Dioxan wurden 30 mg (0,028 mmol) Rh₆(CO)₁₆ und 0,66 g (6,6 mmol) Triethylamin vorgelegt. Es wurden 39,1 mmol Acetylen aufgepreßt, wobei sich ein Druck von 3,4 bar einstellte. Mit Kohlenmonoxid wurde der Druck dann auf 100 bar, mit Wasserstoff auf 200 bar gesteigert. Der Ansatz wurde 5h bei 120°C gerührt. Nach Entspannen des Reaktors und destillativer Aufarbeitung wurde Butyrolacton in 82% Ausbeute isoliert.

### Beispiel 2 (Verfahren A)

Die Reaktion wurde analog zu Beispiel 1 ausgeführt, jedoch bei einer Temperatur von 80°C. Die Ausbeute an Butyrolacton betrug 67%.

### Beispiel 3 (Verfahren A)

In 65 ml Dioxan wurden 30 mg (0,028 mmol) Rh₆(CO)₁₆, 0,66 g (6,6 mmol) Triethylamin, 40 mg (0,32 mmol) Hydrochinonmonomethylether und 12,5 mg (0,08 mmol) NaI vorgelegt. Es wurden 39,1 mmol Acetylen aufgepreßt, wobei sich ein Druck von 3,4 bar einstellte. Mit Kohlenoxid wurde der Druck dann auf 100 bar und mit Wasserstoff auf 200 bar gesteigert. Der Ansatz wurde 1,5 h bei 100°C gerührt. Nach Entspannen des Reaktors und destillativer Aufarbeitung wurde Butyrolacton in 84 % Ausbeute isoliert.

### Beispiel 4 (Verfahren B)

In 65 ml Dioxan wurden 30 mg (0,028 mmol) Rh₆(CO)₁₆, 0,66 g (6,6 mmol) Triethylamin, 4,48 g Wasser, 40 mg (0,32 mmol) Hydrochinonmonomethylether und 12,5 mg (0,08 mmol) NaI vorgelegt. Es wurden 39,5 mmol Acetylen aufgepreßt, wobei sich ein Druck von 3,5 bar einstellte. Mit Kohlenoxid wurde der Druck auf 200 bar gesteigert. Der Ansatz wurde 1,5 h bei 150°C gerührt. Nach Entspannen des Reaktors und destillativer Aufarbeitung wurde Butyrolacton in 71 % Ausbeute isoliert.

### Beispiel 5 (Verfahren B)

In 65 ml Dioxan wurden 30 mg (0,028 mmol) Rh₆(CO)₁₆, 0,66 g (6,6 mmol) Triethylamin, 4,48 g (249 mmol) Wasser, 40 mg (0,36 mmol) Hydrochinonmonomethylether und 12,5 mg (0,08 mmol) NaI vorgelegt. Es wurden 56,6 mmol Acetylen aufgepreßt, wobei sich ein Druck von 5,9 bar einstellte. Mit Kohlenoxid wurde der Druck dann auf 100 bar gesteigert. Der Ansatz wurde 2 h bei 80°C gerührt. Nach Entspannen des Reaktors und destillativer Aufarbeitung wurde 2-(5H)-Furanon in 84 % Ausbeute isoliert.

### Beispiel 6 (Verfahren B)

In 65 ml Dioxan wurden 30 mg (0,028 mmol) Rh₆(CO)₁₆, 0,66 g (6,6 mmol) Triethylamin, 4,48 g (249 mmol) Wasser, 21 mg (0,169 mmol) 2,4,6-Trimethylpyridin und 12,5 mg (0,08 mmol) NaI vorgelegt. Es wurden 57 mmol Acetylen aufgepreßt, wobei sich ein Druck von 6,0 bar einstellte. Mit Kohlenoxid wurde der Druck dann auf 100 bar gesteigert. Der Ansatz wurde 2 h bei 80°C gerührt. Nach Entspannen des Reaktors und destillativer Aufarbeitung wurde 2-(5H)-Furanon in 86 % Ausbeute isoliert.

### Beispiel 7 (Verfahren A)

In 65 ml Dioxan wurden 30 mg (0,028 mmol) Rh₆(CO)₁₆, 0,66 g (6,6 mmol) Triethylamin und 12,5 mg (0,08 mmol) NaI vorgelegt. Es wurden 57 mmol Acetylen aufgepreßt, wobei sich ein Druck von 5,8 bar einstellte. Mit Kohlenoxid wurde der Druck auf 80 bar und dann mit Wasserstoff auf 200 bar gesteigert. Der Ansatz wurde 1 h bei 100°C gerührt. Nach Entspannen des Reaktors und destillativer Aufarbeitung wurde 2-(5H)-Furanon in 67 % Ausbeute isoliert.

### Beispiel 8 (Hydrierung von 2(5H)-Furanon)

In einem 250 ml-Rührkolben, der mit einem Begasungsrührer und einer Gasbürette ausgestattet war, wurden 5,0 g des nach dem erfindungsgemäßen Verfahrens hergestellten und vom Katalysator abgetrennten 2(5H)-Furanons zusammen mit 100 ml Dioxan und 0,5 g eines Hydrierkatalysators (5 Gew.-% Pd auf Kohlensotff) vorgelegt.

Die Lösung wurde bei Raumtemperatur und Normaldruck mit Wasserstoff begast. Nach 30 min war die Wasserstoffaufnahme beendet. Nach Entfernung von Katalysator und Lösungsmittel wurde γ-Butyrolacton mit einer Ausbeute von 96 % isoliert.

## Patentansprüche

1. Verfahren zur Herstellung von Butyrolactonen der allgemeinen Formel I 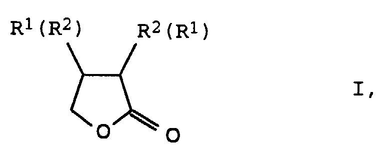 in der die Substituenten R¹ und R² für Wasserstoff, Alkyl-, Hydroxyalkyl-, gegebenenfalls inerte Substituenten tragende Aryl- und Trialkylsilylgruppen stehen, dadurch gekennzeichnet, daß man ein Alkin der allgemeinen Formel II
R¹―C≡C―R² II,
in der die Substituenten die oben angegebene Bedeutung haben, mit Kohlenmonoxid und Wasserstoffgas oder in situ gebildetem Wasserstoff in Gegenwart eines Übergangsmetallkatalysators unter erhöhtem Druck und erhöhter Temperatur umsetzt.

2. Verfahren gemäß Anspruch 1, dadurch gekennzeichnet, daß man den in situ gebildeten Wasserstoff mit CO und Wasser über das Wassergasgleichgewicht erzeugt.

3. Verfahren nach Anspruch 1, dadurch gekennzeichnet, daß man die Umsetzung in Gegenwart eines Amins oder eines Ammoniumsalzes durchführt.

4. Verfahren nach Anspruch 1, dadurch gekennzeichnet, daß man Rhodium- oder Rutheniumverbindungen als Katalysator einsetzt.

5. Verfahren nach Anspruch 1, dadurch gekennzeichnet, daß man eine Rhodiumcarbonylverbindung als Übergangsmetallkatlysator verwendet.

6. Verfahren nach Anspruch 1, dadurch gekennzeichnet, daß man Acetylen als Alkin der Formel II verwendet.

7. Verfahren nach Anspruch 1, dadurch gekennzeichnet, daß man mindestens 2 Äquivalente CO und 2 Äquivalente Wasserstoff H₂ je Äquivalent Alkin der Formel II bei einem Reaktionsdruck von 170 bis 280 bar verwendet.

8. Verfahren nach Anspruch 1, dadurch gekennzeichnet, daß man mindestens 4 Äquivalente CO und 2 Äquivalente Wasser je Äquivalent Alkin der allgemeinen Formel II bei einem Reaktionsdruck von 70 bis 280 bar verwendet.

9. Verfahren nach Anspruch 1, dadurch gekennzeichnet, daß man die Umsetzung in Gegenwart eines Halogenids durchführt.

10. Verfahren nach Anspruch 1, dadurch gekennzeichnet, daß man die Umsetzung in Gegenwart eines Jodids durchführt.

11. Verfahren nach Anspruch 1, dadurch gekennzeichnet, daß man die Umsetzung in Gegenwart eines Polymerisationsinhibitors durchführt.

12. Verfahren zur Herstellung von 2-(5H)-Furanonen der allgemeinen Formel III 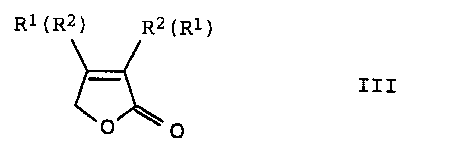 in der die Substituenten R¹ und R² für Wasserstoff, Alkyl, Hydroxylalkyl-, gegebenenfalls inerte Substituenten tragende Aryl- oder Trialkylsilylgruppen stehen, dadurch gekennzeichnet, daß man Alkine der allgemeinen Formel II
R¹―C≡C―R² II,
in denen R¹ und R² die oben genannte Bedeutung haben, mit Kohlenmonoxid und Wasserstoffgas in Gegenwart von Übergangsmetall-Katalysatoren und Aminbasen bei Temperaturen von 60 - 140°C und einem Wasserstoffpartialdruck von mehr als 50 bar umsetzt.

13. Verfahren gemäß Anspruch 1, dadurch gekennzeichnet, daß man gemäß dem Verfahren nach Anspruch 12 zunächst 2-(5H)-Furanone der allgemeinen Formel III 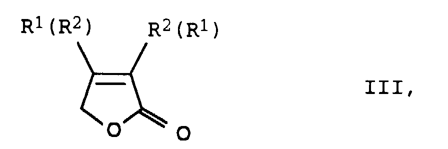 in der R¹ und R² die in Anspruch 12 angegebenen Bedeutungen haben, herstellt und diese in einer getrennten Hydrierungsreaktion zu den entsprechenden Butyrolactonen hydriert.

14. Verfahren nach Anspruch 13, dadurch gekennzeichnet, daß man von Acetylen ausgeht und Butyrolacton herstellt.

## Claims

1. A process for the preparation of butyrolactones of the formula I 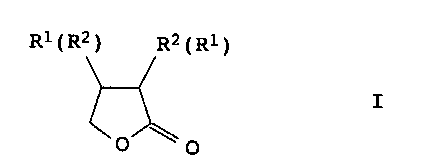 where the substituents R¹ and R² are hydrogen, alkyl or hydroxyalkyl groups, or aryl and trialkylsilyl groups, if appropriate carrying inert substituents, which comprises reacting an alkyne of the formula II
R¹―C≡C―R² II
where the substituents have the meanings indicated above, with carbon monoxide and hydrogen gas or with hydrogen formed in situ in the presence of a transition metal catalyst at elevated pressure and elevated temperature.

2. A process as claimed in claim 1, wherein the hydrogen formed in situ is generated by means of the water-gas equilibrium using CO and water.

3. A process as claimed in claim 1, wherein the reaction is carried out in the presence of an amine or of an ammonium salt.

4. A process as claimed in claim 1, wherein rhodium or ruthenium compounds are used as catalyst.

5. A process as claimed in claim 1, wherein a rhodium carbonyl compound is used as transition metal catalyst.

6. A process as claimed in claim 1, wherein acetylene is used as alkyne of the formula II.

7. A process as claimed in claim 1, wherein at least 2 equivalents of CO and 2 equivalents of hydrogen H₂ are used per equivalent of alkyne of the formula II at a reaction pressure of from 170 to 280 bar.

8. A process as claimed in claim 1, wherein at least 4 equivalents of CO and 2 equivalents of water are used per equivalent of alkyne of the formula II at a reaction pressure of from 70 to 280 bar.

9. A process as claimed in claim 1, wherein the reaction is carried out in the presence of a halide.

10. A process as claimed in claim 1, wherein the reaction is carried out in the presence of an iodide.

11. A process as claimed in claim 1, wherein the reaction is carried out in the presence of a polymerization inhibitor.

12. A process for the preparation of 2-(5H)-furanones of the formula III 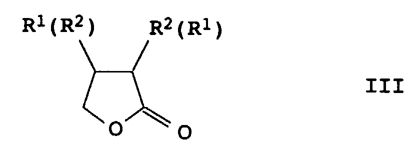 where the substituents R¹ and R² are hydrogen, alkyl or hydroxyalkyl groups, or aryl or trialkylsilyl groups, if appropriate carrying inert substituents, which comprises reacting alkynes of the formula II
R¹―C≡C―R² II
where R¹ and R² have the abovementioned meanings, with carbon monoxide and hydrogen gas in the presence of transition metal catalysts and amine bases at 60-140°C and a hydrogen partial pressure of more than 50 bar.

13. A process as claimed in claim 1, wherein 2-(5H)-furanones of the formula III 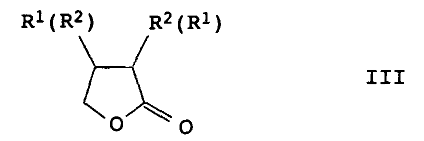 where R¹ and R² have the meanings indicated in claim 12, are first prepared by the process as claimed in claim 12 and these are hydrogenated to the corresponding butyrolactones in a separate hydrogenation reaction.

14. A process as claimed in claim 13, wherein acetylene is used as a starting material and butyrolactone is prepared.

## Revendications

1. Procédé de préparation de butyrolactones de formule générale I 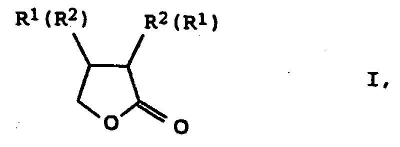 dans laquelle les substituants R¹ et R² sont mis pour des atomes d'hydrogène, pour des groupements alkyle, hydroxyalkyle, aryle et trialkylsilyle portant éventuellement des substituants inertes, caractérisé en ce que l'on fait réagir un alcyne de formule générale II
R¹―C≡C―R² II,
dans laquelle les substituants ont la signification mentionnée ci-dessus, avec du monoxyde de carbone et de l'hydrogène gazeux ou de l'hydrogène formé in situ, en présence d'un catalyseur à base de métaux de transition, sous pression élevée et température élevée.

2. Procédé selon la revendication 1, caractérisé en ce que l'on produit l'hydrogène in situ avec du CO et de l'eau au moyen de l'équilibre du gaz à l'eau.

3. Procédé selon la revendication 1, caractérisé en ce que l'on mène la réaction en présence d'une amine ou d'un sel d'ammonium.

4. Procédé selon la revendication 1, caractérisé en ce que l'on utilise des composés du rhodium ou du ruthénium, en tant que catalyseur.

5. Procédé selon la revendication 1, caractérisé en ce que l'on utilise un composé rhodium-carbonyle en tant que catalyseur à base de métaux de transition.

6. Procédé selon la revendication 1, caractérisé en ce que l'on utilise de l'acétylène en tant qu'alcyne de formule II.

7. Procédé selon la revendication 1, caractérisé en ce que l'on utilise au moins deux équivalents de CO et deux équivalents d'hydrogène H₂ par équivalent d'alcyne de formule II, sous une pression réactionnelle de 170 à 280 bar.

8. Procédé selon la revendication 1, caractérisé en ce que l'on utilise au moins quatre équivalents de CO et deux équivalents d'eau par équivalent d'alcyne de formule II, sous une pression réactionnelle de 170 à 280 bar.

9. Procédé selon la revendication 1, caractérisé en ce que l'on mène la réaction en présence d'un halogénure.

10. Procédé selon la revendication 1, caractérisé en ce que l'on mène la réaction en présence d'un iodure.

11. Procédé selon la revendication 1, caractérisé en ce que l'on mène la réaction en présence d'un inhibiteur de polymérisation.

12. Procédé de préparation de 2-(5H)-furanones de formule générale III 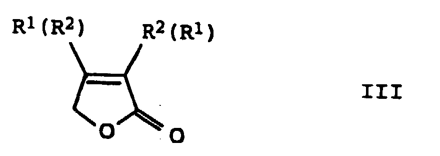 dans laquelle les substituants R¹ et R² sont mis pour des atomes d'hydrogène, pour des groupements alkyle, hydroxyalkyle, aryle et trialkylsilyle portant éventuellement des substituants inertes, caractérisé en ce que l'on fait réagir un alcyne de formule générale II
R¹―C≡C―R² II,
dans laquelle les substituants ont la signification mentionnée ci-dessus, avec du monoxyde de carbone et de l'hydrogène gazeux, en présence d'un catalyseur à base de métaux de transition et de bases aminées, à des températures de 60-140°C et sous une pression partielle en hydrogène de plus de 50 bar.

13. Procédé selon la revendication 1, caractérisé en ce que l'on prépare tout d'abord, suivant le procédé selon la revendication 12, des 2-(5H)-furanones de formule générale III 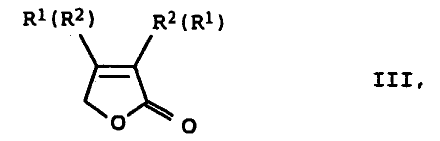 dans laquelle R¹ et R² prennent la signification mentionnée dans la revendication 12, puis on les hydrogène lors d'une étape d'hydrogénation distincte pour obtenir les butyrolactones correspondantes.

14. Procédé selon la revendication 13, caractérisé en ce que l'on part de l'acétylène pour préparer de la butyrolactone.
